# EUROPÄISCHE PATENTSCHRIFT

(11) **EP 0 641 200 B1**
(45) Veröffentlichungstag und Bekanntmachung des Hinweises auf die Patenterteilung: **02.12.1998**
(21) Anmeldenummer: 93912719.7
(22) Anmeldetag: 19.05.1993
(51) Int. Cl.: A61K 31/19, A61K 9/20

(54) **2-ARYLPROPIONSÄURE-ZUBEREITUNGEN UND VERFAHREN ZU IHRER HERSTELLUNG**
2-ARYLPROPIONIC ACID COMPOSITIONS AND PROCESS FOR PREPARING THE SAME
COMPOSITIONS D'ACIDE 2-ARYLPROPIONIQUE ET LEUR PROCEDE DE PREPARATION

(30) Priorität: 21.05.1992 DE 4216756
(43) Veröffentlichungstag der Anmeldung: 08.03.1995
(73) Patentinhaber: PAZ ARZNEIMITTEL- ENTWICKLUNGSGESELLSCHAFT MBH, 65933 Frankfurt (DE)
(72) Erfinder: GEISSLINGER, Gerd, D-8500 Nürnberg (DE); BRUNE, Kay, D-8525 Marloffstein (DE); BAUER, Kurt, D-7800 Freiburg (DE); HUBER, Anton, S., CH-4011 Basel (CH)
(74) Vertreter: Grussdorf, Jürgen, Dr.
(86) Internationale Anmeldenummer: EP9301243
(87) Internationale Veröffentlichungsnummer: WO9323026

(56) Entgegenhaltungen:
- EP-A- 0 241 615
- EP-A- 0 295 212
- US-A- 4 873 231

## Beschreibung

Die Erfindung betrifft neue Zubereitungen von 2-Arylpropionsäuren mit verbesserter Tablettierbarkeit und verbesserter Festigkeit, die insbesondere Ibuprofen-Racemat, S-Ibuprofen oder Mischungen von R- und S-Ibuprofen sowie übliche Hilfs- und/oder Trägerstoffe enthalten sowie Verfahren Zu ihrer Herstellung.

2-Arylpropionsäurederivate wie Ibuprofen, Flurbiprofen, Ketoprofen, Tiaprofen oder Pirprofen sowie deren Enantiomeren werden in der Therapie als nichtsteroidale Antiphlogistika, Antirheumatika, Analgetika oder Antipyretika eingesetzt.

Aus technologischer Sicht besitzen diese Arzneistoffe Nachteile. Sie sind in Wasser nur schlecht löslich und besitzen relativ niedrige Schmelzbereiche. So schmilzt z.B. das Ibuprofen-Racemat bei ca. 75-77°C, das Flurbiprofen-Racemat bei ca. 110-111°C und das Ketoprofen-Racemat bei ca. 94°C. Die Enantiomere des Ibuprofens schmelzen sogar schon bei ca. 50°C.

Stoffe mit niedrigem Schmelzbereich führen bekannterweise bei der Tablettierung infolge von Sintervorgängen und durch Kleben an den Stempeln und Matrizen der Tablettenpressen zu mehr oder weniger stark störenden Produktionsproblemen (H. Sucker, P. Fuchs und P. Speiser: Pharmazeutische Technologie, Georg Thieme Verlag, Stuttgart 1978, S. 381).

Das Kleben dieser niedrigschmelzenden Arzneistoffe kann durch Zumischen großer Mengen von Antiklebmitteln (Formentrennmittel) behoben werden. Dabei werden die Mischungen jedoch hydrophob. Daraus wiederum resultiert eine verlangsamte Freisetzung des Arzneistoffes, verbunden mit einer verlangsamten Resorption oder sogar schlechte Bioverfügbarbeiten. Die Tabletten können auch infolge Überdosierung der Antiklebmittel zu weich werden.

Bis zu einem bestimmten Grad lassen sich die Komplikationen bei der Tablettenherstellung durch die Zugabe von bestimmten Tablettenhilfsstoffen, wie Antiklebmittel bzw. Schmiermittel, in höheren Dosen sowie durch eine drastische Reduktion der Preßgeschwindigkeit beheben. Darüber hinaus ist erfahrungsgemäß bei Tabletten, die niedrigschmelzende Ingredientien enthalten, nach bestimmten Lagerzeiten infolge von Sinterungen mit Nachhärtungen zu rechnen (K.H. Bauer, K.H. Frömming und C. Führer: Pharmazeutische Technologie, Georg Thieme Verlag, Stuttgart, New York, 1986). Diese Nachhärtungen ziehen Verschlechterungen der Zerfallszeit und nicht ausreichende Arzneistoff-Freisetzungen bzw. nicht ausreichende Bioverfügbarkeiten nach sich.

Bisher wurde eine Tablettierung von 2-Arylpropionsäuren mit wechselndem Erfolg versucht, durch Zusatz von Formentrenn- oder Schmiermitteln, Trockenmitteln (z.B. hochdisperse Kieselsäure), geeigneten Füllmitteln und von starken Zerfallsbeschleunigern (z.B. Kollidon CL®, querververnetztes PVP), vor allem jedoch durch überdurchschnittliche Erhöhungen der Zusatzmengen dieser Hilfsstoffe, diese Nachteile zu beheben.

In der Europäischen Patentanmeldung EP-A-0 267 321 ist ein Ibuprofen enthaltendes Arzneimittel beschrieben, welches Ibuprofen nur in der S(+)-Form enthält. Dieses bekannte Arzneimittel besitzt jedoch die oben genannten Nachteile, d.h. es läßt sich nur schlecht tablettieren.

Das Zusammensintern oder -schmelzen kann auch durch Zusatz von größeren Mengen an Füllmittel, zum Beispiel Cellulosepulver oder Lactose, oder durch Sprengmittel, zum Beispiel Stärke, vermindert werden. Zu hohe Zusätze von Sprengmittel haben jedoch meist zu weiche Tabletten mit erhöhter Friabilität zur Folge, da sich Stärke oder andere ähnlich elastische Stoffe allein oder in hoher Dosierung nur schlecht komprimieren lassen oder die Tabletten werden so groß, daß sie nur schwer zu schlucken sind.

In der Deutschen Patentanmeldung P 39 22 441.4 ist ein Verfahren beschrieben, die Tablettierbarkeit von Ibuprofen und S(+)-Ibuprofen zu verbessern. Es wurde gefunden, daß, wenn Ibuprofen oder S(+)-Ibuprofen ganz oder teilweise in ihre Calciumsalze überführt werden und diese zur Tablettenherstellung eingesetzt werden, die Tablettierbarkeit deutlich verbessert werden kann. Bereits 25 % Calciumsalz des entsprechenden Ibuprofens verbessern die Tablettiereigenschaften merklich, bevorzugt werden jedoch 50 bis 100 %. Weiterhin kann der übrige Teil des Ibuprofens in die ebenfalls höherschmelzenden Natrium- oder Ammoniumsalze überführt werden. Die Überführung des Ibuprofens in das Calciumsalz erfolgt durch Umsetzen mit einer wässrigen Lösung von Ca(OH)₂ oder einem löslichen Calciumsalz. Vorzugsweise wird diese Umsetzung beim Granulationsprozeß mit durchgeführt. Dieses Verfahren zeigt zwei Nachteile:
1. Die Überführung des Ibuprofens in das Calciumsalz während der Granulierung ist aufwendig und technisch nicht leicht durchführbar. Die erheblichen Mengen an wässriger Lösung müssen abgedampft werden.
2. Verglichen mit anderen Ibuprofensalzen ist Calciumibuprofenat schwer löslich in Wasser. Somit muß, insbesondere wenn der Anteil an Calciumibuprofenat in der Arzneiform sehr hoch ist, mit negativen Eigenschaften hinsichtlich Zerfall, Wirkstoff-Freisetzung und Bioverfügbarkeit gerechnet werden. So werden positive Tablettiereigenschaften mit Nachteilen hinsichtlich der Pharmakokinetik erkauft. Aufgrund der hohen Dosierung in der Grundrezeptur, z.B. des Ca-Na Glycinates, welches in molaren Mengen zugesetzt wird, resultieren sehr harte Tabletten, die teilweise Zerfallszeiten bis zu 3 Stunden zeigten.

Der vorliegenden Erfindung liegt nun die Aufgabe zugrunde, 2-Arylpropionsäuren oder deren Enantiomere, insbesondere eine Ibuprofen, S- oder R-Ibuprofen oder Mischungen aus bei-den Enantiomeren enthaltende Zubereitung mit verbesserter Tablettierbarkeit, verbesserter Festigkeit und verbesserter Freisetzung sowie einfache wirtschaftliche Verfahren zu deren Herstellung zur Verfügung zu stellen.

Diese Aufgabe wird durch die in den Ansprüchen beschriebenen Merkmale gelöst bzw. gefördert.

Überraschenderweise wurde gefunden, daß bereits beim trokkenen Mischen von 100 Gew.-Teilen von Ibuprofen oder anderen 2-Arylpropionsäuren mit ca. 50-500 Gew.-Teilen, vorzugsweise 50-150 Gew.-Teilen, Calciumverbindungen (z.B. CaHPO₄, CaCO₃, Ca(OH)₂ etc.), d.h. ungefähr äquimolaren Mengen, ohne Probleme überraschend gut fließfähige und komplikationslos tablettierbare Pulvergemische entstehen. Besonders gute Tablettiereigenschaften wurden erzielt, wenn als Calciumverbindung CaH(PO₄) eingesetzt wurde. Beim trockenen Zusammenmischen von z.B. Ibuprofen mit anderen in der Tablettierung üblicherweise verwendeten Hilfsstoffen (z.B. mikrokristalline Cellulose, siehe EP-A-0 267 321, Seite 4, Beispiel 1) ist dies nicht der Fall (siehe Anlage). Solche Mischungen zeigen die beschriebenen Nachteile bei der Tablettierung. Die Unterschiede im Fließverhalten Zeigen sich an den unterschiedlichen Böschungswinkeln des Pulvers von 23-27° bei erfindungsgemäßen Produkten gegenüber 38-40° bei Mischungen z.B. mit mikrokristalliner Cellulose.

Weiterhin wurde überraschenderweise gefunden, daß durch den üblichen Zusatz von Mg-stearat oder Ca-stearat, der Versuchsansatz leicht klebrig wurde und somit nur schwer tablettierbar war. Der Anteil an solchen Antiklebmitteln oder Formentrennmitteln oder Schmiermitteln konnte entgegen herkömmlichen Rezepturen, z.B. bei Ibuprofen (EP-A-0 267 321) oder R-Flurbiprofen (DE-A-4028 906), reduziert werden bzw. solche Zusatzstoffe ganz entfallen.

Die vorliegende Erfindung betrifft also ein Verfahren, mit dem Arylpropionsäurederivate oder deren Enantiomere oder Mischungen enthaltende Arzneiformen unter weitgehendem Verzicht auf Schmiermittel oder Formentrennmittel, welche sich negativ auf die galenischen oder pharmakokinetischen Eigenschaften der Formulierung auswirken, hergestellt werden kann.

Bei der trockenen Vermischung und Tablettierung von 2-Arylpropionsäuren mit den erfindungsgemäßen Calciumverbindungen entstehen im Gegensatz zu dem Verfahren der Feuchtgranulierung gemäß DE-A-3 922 441.4 die schwerlöslichen Calciumsalze nur in geringer Menge, vorzugsweise 0.1 - 5 %, so daß die Löslichkeit und demnach die Freisetzungsgeschwindigkeit der Arylpropionsäure nicht verringert wird. Dies läßt sich leicht durch Extraktion der Tabletten mit organischen Lösungsmitteln, in denen die freie Propionsäure, nicht aber die entsprechenden Calciumsalze löslich sind, beweisen.

Bevorzugte Zubereitungen enthalten weiterhin ein Netzmittel, wie z.B. Na-Laurylsulfat, Tween, Na-Dioctylsulfosuccinat, oder ein anderes übliches Netzmittel in niedriger Dosierung, bevorzugt werden 0.1 bis 3 %, wodurch die Freisetzungsrate verbessert wird. Darüber hinaus können natürlich zusätzlich übliche Hilfs- und/oder Trägerstoffe enthalten sein.

Die nachfolgenden Beispiele beschreiben einige erfindungsgemäße Rezepturen ohne die Erfindung zu beschränken.

### Beispiel 1:

### S-Ibuprofen Oblong-Tabletten

1 Tablette enthält:

| | |
|---|---|
| S-Ibuprofen (ca. 99 % optische Reinheit) | 300 mg |
| Emcompress (CaH(PO₄) | 240 mg |
| Explotab (Sprengmittel) | 14 mg |
| Aerosil R 972 (Kieselgel) | 6 mg |

Die Substanzen werden vorgelegt und trocken zusammengeführt und anschließend durch ein z.B. 0.8 mm Sieb geschlagen und auf einer üblichen Matrizen-Tablettenpresse verpreßt. Weder in den Förderleitungen noch in den Matrizen wird ein Klumpen oder Anbacken der Masse beobachtet. Die Tabletten weisen bei guter Härte einen raschen Zerfall im Wasser und eine schnelle Freisetzung des Wirkstoffes auf.

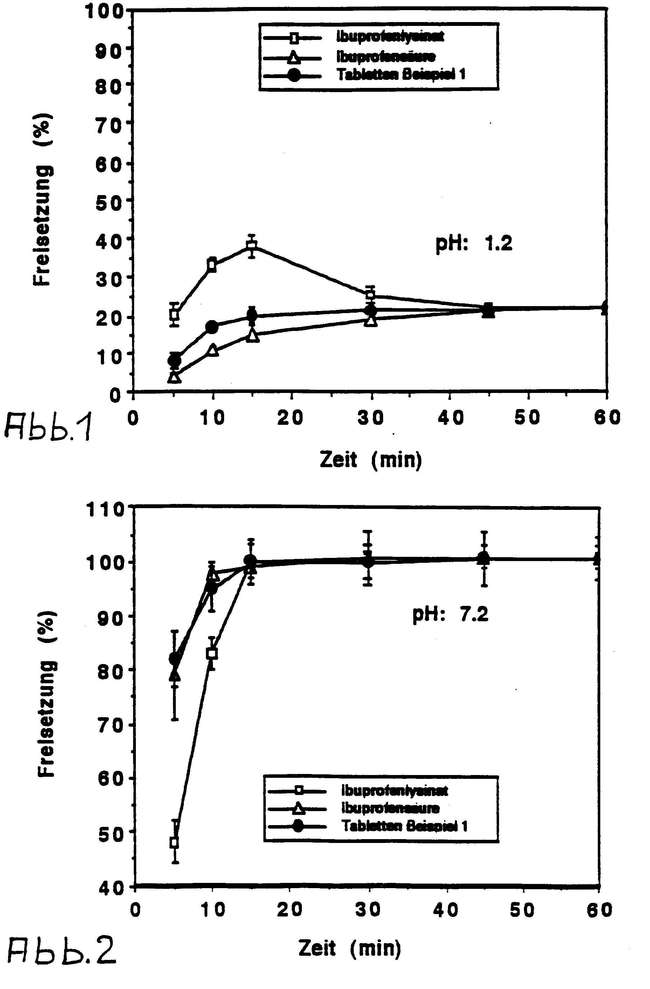

In den Abb. 1 und 2 ist die in vitro Freisetzung der erfindungsgemäßen Tablette mit 2 handelsüblichen Rezepturen verglichen, welche Ibuprofenlysinat bzw. Ibuprofensäure und bekannte Hilfs- und Trägerstoffe enthalten.

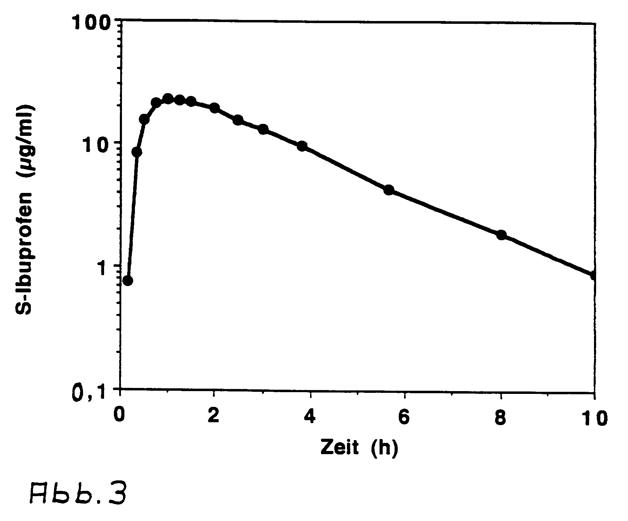

In Abb. 3 ist der Plasma-Konzentrations/Zeitverlauf von S-Ibuprofen nach oraler Gabe einer erfindungsgemäßen Tablette dargestellt. Die Kurve zeigt, daß diese Tabletten den pharmakokinetischen Anforderungen entspricht.

### Beispiel 2:

### S- und R-Ibuprofen Tabletten

1 Tablette enthält:

| | |
|---|---|
| S--Ibuprofen (ca. 99 % optische Reinheit) | 100 mg |
| R--Ibuprofen (ca. 99 % optische Reinheit) | 50 mg |
| Emcompress | 140 mg |
| Explotab | 9 mg |
| Aerosil R 972 | 3 mg |

Herstellung und Eigenschaften wie Beispiel 1.

### Beispiel 3:

### Ibuprofen-Racemat Tabletten

1 Tablette enthält:

| | |
|---|---|
| Ibuprofen Racemat | 300 mg |
| CaCO₃ | 210 mg |
| Explotab | 14 mg |
| Aerosil R 972 | 6 mg |
| Na-Laurylsulfat | 3 mg |

Herstellung wie Beispiel 1.

### Beispiel 4:

### Ketoprofen Kapseln

1 Kapsel enthält:

| | |
|---|---|
| S-Ketoprofen (ca. 95 % optische Reinheit) | 50 mg |
| CaHPO₄ | 40 mg |
| Tween | 1 mg |
| Aerosil | 1 mg |

Abfüllung der trocken gemischten Pulver in Hartgelatine-Kapseln.

### Beispiel 5:

### Flurbiprofen Dragees:

1 Dragee enthält:

| | |
|---|---|
| R-Flurbiprofen (ca. 98 % optische Reinheit) | 50 mg |
| Emcompress | 40 mg |
| Explotab | 4 mg |
| Aerosil R 972 | 2 mg |

Herstellung analog Beispiel 1.

Die Kerne werden anschließend nach bekannten galenischen Rezepturen mit einer Zuckerlösung zum Dragee überzogen.

## Patentansprüche

1. Verfahren zur Herstellung von Zubereitungen aus 2-Arylpropionsäurederivaten mit verbesserter Tablettierbarkeit, welche an sich bekannte Hilfs- und/oder Trägerstoffe enthalten, **dadurch gekennzeichnet**, daß die Arylpropionsäure mit einer Calciumverbindung trocken gemischt wird, und nach Zusatz der übrigen Hilfs- und/oder Trägerstoffe direkt zu Tabletten verpreßt wird.

2. Verfahren nach Anspruch 1, **dadurch gekennzeichnet**, daß als Hilfsstoff ein pharmazeutisch verträgliches Netzmittel eingesetzt wird.

3. Verfahren nach Anspruch 1 oder 2, **dadurch gekennzeichnet**, daß Ibuprofen-Racemat oder S-Ibuprofen als Arylpropionsäurederivat verwendet wird.

4. Verfahren nach Anspruch 1, 2 oder 3, **dadurch gekennzeichnet**, daß als Calciumverbindung CaHPO₄ oder CaCO₃ enthalten ist.

5. Pharmazeutische Zubereitung hergestellt gemäß einem der Ansprüche 1 bis 4.

## Claims

1. Process for the production of compositions of 2-arylpropionic acid derivatives with improved tablettability which contain per se known adjuvant and/or carrier materials, characterised in that the arylpropionic acid is mixed in the dry state with a calcium compound and, after addition of the remaining adjuvant and/or carrier materials, pressed directly to tablets.

2. Process according to claim 1, characterised in that a pharmaceutically compatible wetting agent is used as adjuvant material.

3. Process according to claim 1 or 2, characterised in that ibuprofen racemate or S-ibuprofen is used as arylpropionic acid derivative.

4. Process according to claim 1, 2 or 3, characterised in that CaHPO₄ or CaCO₃ is contained as calcium compound.

5. Pharmaceutical composition according to one of claims 1 to 4.

## Revendications

1. Procédé pour la préparation de formulations constituées de dérivés d'acide 2-arylpropionique avec une aptitude améliorée au pastillage, qui contiennent des excipients et/ou supports connus, caractérisé en ce que l'acide arylpropionique est mélangé à sec avec un composé du calcium et pastillé directement sous forme de comprimés après l'addition des autres excipients et supports.

2. Procédé selon la revendication 1, caractérisé en ce qu'on utilise à titre d'excipient un agent mouillant pharmaceutiquement acceptable.

3. Procédé selon la revendication 1 ou 2, caractérisé en ce qu'on utilise le racémate d'ibuprofène ou le S-ibuprofène à titre de dérivé d'acide arylpropionique.

4. Procédé selon la revendication 1, 2 ou 3, caractérisé en ce que du CaHPO₄ ou du CaCO₃ y est contenu à titre de composé du calcium.

5. Formulation pharmaceutique préparée selon l'une des revendications 1 à 4.
